# EUROPEAN PATENT SPECIFICATION

(11) **EP 1 861 063 B1**
(45) Date of publication and mention of the grant of the patent: **18.01.2012**
(21) Application number: 06723555.6
(22) Date of filing: 20.03.2006
(51) Int. Cl.: A61K 8/04, A61Q 17/04

(54) **Polysiloxane coated metal oxide particles**
Polysiloxanbeschichtete Metalloxidpartikel
Particules d'oxyde métallique enduites de polysiloxane

(30) Priority: 23.03.2005 EP 05006450
(43) Date of publication of application: 05.12.2007
(73) Proprietor: DSM IP Assets B.V., 6411 TE Heerlen (NL)
(72) Inventor: BERG-SCHULTZ, Katja, CH-4303 Kaiseraugst (CH); VOLLHARDT, Juergen, H., CH-4433 Ramlinsburg (CH); WESTENFELDER, Horst, 67435 Neustadt A.d.W. (DE); SIT, Fintan, Singapore 229813 (SG)
(74) Representative: Best, Michael
(86) International application number: PCT/EP2006/002537
(87) International publication number: WO 2006/100018

(56) References cited:
- EP-A- 0 709 080
- EP-A- 0 748 624
- WO-A-93/04665
- DSM PRESS RELEASE, 8 September 2004 (2004-09-08), XP002385513 Retrieved from the Internet: URL:http://www.dsm.com/en_US/html/dnp/news _rel_2004_08.htm> [retrieved on 2006-06-14]

## Description

The present invention relates to novel UV filters, a process for the preparation and their use especially in formulations for the protection of human skin and/ or hair against harmful effects of sunlight. The novel UV filters are metal oxide particles which are coated with sunscreens on the basis of polysiloxanes.

There is a constantly increasing need for sunscreen protection agents in a population that is exposed to an increasing amount of damaging sunlight. Repetitive sun exposure can result in skin changes known as photoaged skin. The clinical changes that are seen in photoaged skin differ from those of normally aged skin in sunlight protected sites of the body. Among the damaging results of intensive sun exposure of the skin there is increased wrinkling, elastosis, pigmentary changes, precancerous and cancerous skin lesions.

Many sunscreening chemicals have been developed in the past protecting against the harmful effect of UV-A (320 to 400 nm) or UV-B (290-320 nm) wavelength, UV-A and UV-B (290 to 400 nm) wavelength and even shorter wavelength (UV-C). Very recently a new class of sun screening chemicals, the broadband UV-filters have been developed which shield the skin from UV-A and UV-B radiation. These chemicals are usually incorporated either alone or in combination with each other into cosmetic or pharmaceutical preparations which are widely known and used.

Most UV filters used in sunscreen compositions are monomeric compounds, and thus there is the inherent risk that such compounds can penetrate the skin barrier, which is highly undesirable. UV filters on the basis of polysiloxanes which may be either linear or cyclic are described e.g. in WO 93/04665, WO 94/06404, EP-A 538 431, EP-A 392 883 and EP-A 358 584. With these polysiloxanes the risk of skin penetration is lower, but it is sometimes difficult to incorporate the polysiloxanes in sunscreen compositions due to incompatibility problems which differ depending on the UV-active chromophores which are covalently bonded to the polysiloxanes.

Apart from carbon-based chromophores metal oxides such as titanium dioxide or zinc oxide are widely used in sunscreening agents. Their action is substantially based on the reflection, scattering and absorption of harmful UV radiation and is substantially dependent upon the primary particle size of the metal oxide.

However, incorporation of metal oxide in sunscreen compositions can cause significant problems, in particular because many metal oxides such as titanium dioxide or zinc oxide tend to agglomerate in many finished formulations leading to a loss in their efficacy as sunscreening agent and resulting in an unacceptable aesthetic appearance due to a so-called 'whitening effect' when applied to the skin. Furthermore, the metal oxides such as titanium dioxide or zinc oxide show formulation incompatibilities when used in combination with other usual ingredients of cosmetic or dermatological formulations and/ or other UV-sunscreens. Especially unwanted are incompatibilities with other UV-sunscreens such as dibenzoylmethane derivatives (e.g. 4,4'-Methoxy-tert.butyldibenzoylmethane) resulting amongst others e.g. in a coloration of the formulation which is highly undesirable. Additionally, untreated metal oxides are known to have photocatalytic activity through which reactions are triggered which may lead to changes of components of sunscreening agents or cause skin irritations.

Various coatings have been proposed for modifying the surface of the metal oxide in order to ease the incorporation into the formulation, to avoid the formulation incompatibilities and to reduce the photocatalytic activity such as e.g. simethicone, methicone and triethoxy caprylylsilane and silicon dioxide e.g. described in EP1 281 388 and DE 103 33 029. In particular in view of their photocatalytic activity it has been suggested to coat the metal oxide particles with an inorganic coating which is not degenerated by a photocatalytic reaction. Coatings on the basis of calcinated alumina and of other oxides are known. EP-A 988 853, EP-A 1 284 277, EP0988853, and US 5562897 disclose silica coated metal oxide powders. EP-A 748 624 discloses polysiloxane and octyl methylsilane coated TiO₂ particles. While these coatings solve some problems associated with the use of metal oxide particles as UV absorbing or scattering particles in sunscreen compositions, there is still need for improved coated metal oxide particles, in particular for UV absorbing and/or scattering particles which allow a wide variability regarding the range of UV light which they absorb or scatter while nevertheless avoid the manifold problems occurring after incorporation of those UV absorbing particles into cosmetic or dermatological compositions. The UV filters of this invention should not show the shortcomings of the prior art UV filters, They should have excellent UV filter activity, being easily accessible and compatible with the other usual ingredients of cosmetic and dermatological compositions and with other UV-sunscreens e.g. such as dibenzoylmethane derivatives.

For solving these problems the present invention for the first time provides novel UV filter particles which combine both the advantages of coated metal oxide particles and the advantages of chromophores on carbon basis which have excellent UV filter activity over a broad wavelength independent of the particle size. With these novel UV filter particles sunscreen formulations with high SPF values can be generated while avoiding the disadvantages of the prior art metal oxide particles and the prior art chromophores on carbon basis. Surprisingly, it was also found that the coated metal oxides of this invention additionally stabilize dibenzoylmethane derivatives such as 4,4'-Methoxy-tert.butyldibenzoylmethane against degradation upon irradiation. Such dibenzoyl methane derivatives are well known to have a limited photostability.

The present invention therefore provides novel coated metal oxide particles comprising a metal oxide particle and at least one coating, wherein the at least one coating comprises a. The present invention also provides a process for producing these coated metal oxide particles. The sunscreens on polysiloxane basis have UV-A and/or UV-B and/or UV-C filter and/or broadband filter activity and can be prepared by state of the art methods. The present invention also provides coated metal oxide particles obtainable by this process, sunscreen compositions comprising those coated metal oxide particles and the use of the coated metal oxide particles for producing cosmetic and dermatological compositions, especially sunscreen compositions. Additionally, the present invention provides a method for the stabilization of dibenzoylmethane derivatives against degradation upon irradiation.

The sunscreens on polysiloxane basis used for the coating may be either linear or cyclic and are described e.g. in WO 93/04665, WO 94/06404, WO 92/20690, EP-A 392 883, EP 538 431, EP 358 584, WO 03/035 022, EP-A 709 080, WO2004007592, and in WO 03/086340. Preferred are the polysiloxane sunscreens which are disclosed in WO 03/035 022, WO03/030 856 and EP-A 709 080 and reference is explicitly made to the definition of the polysiloxane sunscreens as used in these documents. The preferred polysiloxane sunscreens disclosed in WO 03/035 022, WO03/030 856 and EP-A 709 080 are also the preferred polysiloxane sunscreens of the present application.

The term sunscreen on polysiloxane basis (or polysiloxane sunscreen) as used herein generally denotes a siloxane, particularly a polysiloxane which carries at least one group which absorbs UV-A and/or UV-B radiation. Examples of such siloxane sunscreens are those of the general formula 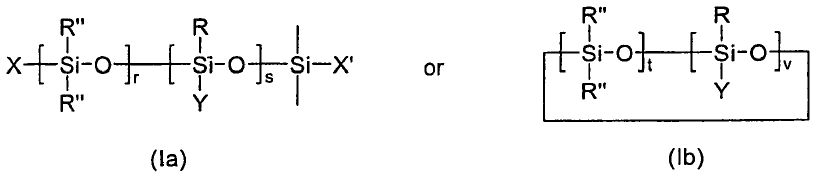 wherein R and R" are C₁₋₁₀-alkyl or phenyl;
X and X' are C₁₋₁₀-alkyl, phenyl or a group Y;
r is an integer from 0 to 150;
s is an integer from 0 to 30; where, when s = 0 at least one of X and X' is Y; the sum of r and s is ≥ 3;
t is an integer from 0 to 10;
v is an integer from 0 to 10;
the sum of v and t is ≥ 3;
Y is a UV-A or UV-B -light absorbing chromophore,

Preferably Y is a group of the formulas (a) and/ or (b) 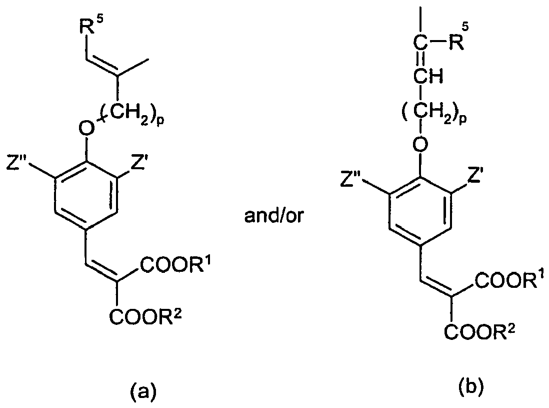 wherein
R¹ and R² are C₁₋₆-alkyl;
R⁵ is hydrogen or C₁₋₆-alkyl;
Z' and Z" are, independently, hydrogen, hydroxy, C₁₋₆-alkyl or C₁₋₆-alkoxy; and
p is an integer from 1 to 6;
or a group of the formula (c) 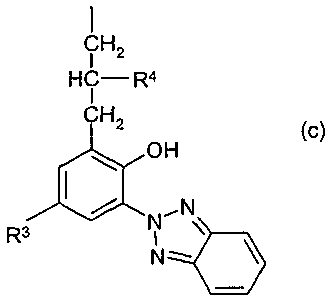 wherein R³, R⁴ is hydrogen or C₁₋₆-alkyl.

Compounds of the formula Ia and Ib wherein Y is a group (a) and/or (b) and their preparation are disclosed, e.g., in EP 0 538 431 A and EP 0 679 645 A. Compounds of the formula la and Ib wherein Y is a group (c) and their preparation are disclosed, e.g., in EP 0 354 145, EP 0 388 218, EP 0 392 883 and WO 94/06404. Compounds with v = 0 are not preferred, v is preferably 1 to 10.

In the above formulas (Ia) and (Ib) it is to be understood that the residues 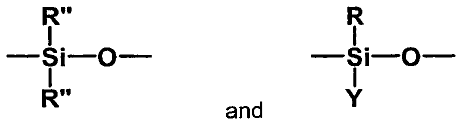 can be (and preferably are) statistically distributed in the polymer. The formula (Ia), respectively (Ib), should thus not be interpreted that the polymer necessarily contains a block of r (or t) residues 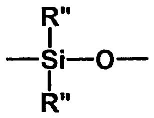 and a block of s, (or v) residues 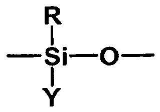 but that the polymer contains r, (or t) residues 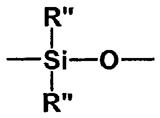 and s, (or v) residues 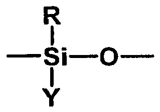 which can be statistically distributed. Linear polysiloxane compounds wherein the chromophore carrying residue is statistically distributed are preferred. Said preferred polysiloxane compounds have at least one unit carrying the chromophore residue (s=1), preferably s has a value of from about 2 to about 10, more preferably a statistical mean of about 4. The number of r of the other silicone units present in the polysiloxane compounds is preferably about 5 to about 150, more preferably a statistical mean of about 60.

Polysiloxane compounds wherein 20% or less, preferably less than 10%, of the total siloxane units are units carrying a chromophore residue are preferred with respect to cosmetic properties.

The ratio of polysiloxane units having a chromophore residue of the formula a to those having a chromophore residue of the formula b is not critical. Said.ratio may be about 1:1 to about 19:1, preferably about 2:1 to about 9:1, more preferably about 4:1.

The preferred sunscreen on polysiloxane basis for use in the present invention are those of formula la above wherein Y is a group of the formulas (a) and/or (b), particularly those wherein R¹ and R² are ethyl, R⁵, Z' and Z" are hydrogen, and p is 1.

Most preferred are sunscreen on polysiloxane basis of the formula la wherein s is ca. 4 and r is ca. 60, and wherein about 80 % of the groups Y are of the alkylidene structure (a) and about 20 % of the groups Y are of the alkylene structure (b).

These most preferred sunscreen on polysiloxane basis is commercially available under the tradename PARSOL SLX^{®} by DSM (INCI name: polysilicone-15) with the formula 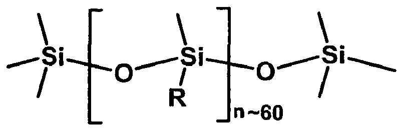 wherein R = CH₃ (approx. 92.1-92.5%)
R= 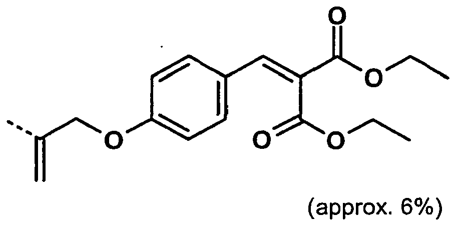 and
R=

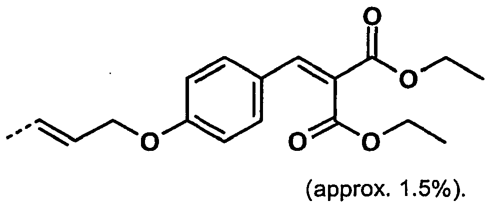

Of the compounds of formula la and Ib wherein Y is a group (c) as defined earlier those wherein R³ and R⁴ are hydrogen or C₁₋₆-alkyl, especially those wherein R³ is methyl and R⁴ is hydrogen or methyl are of primary interest.

The polysiloxane compounds la wherein Y is a reside of the formula a and/or b can be prepared as described in EP-B 0 538 431 by hydrosilylation of a methylhydrosilane-dimethylsiloxane copolymer with the corresponding benzalmalonates in the presence of a catalyst: 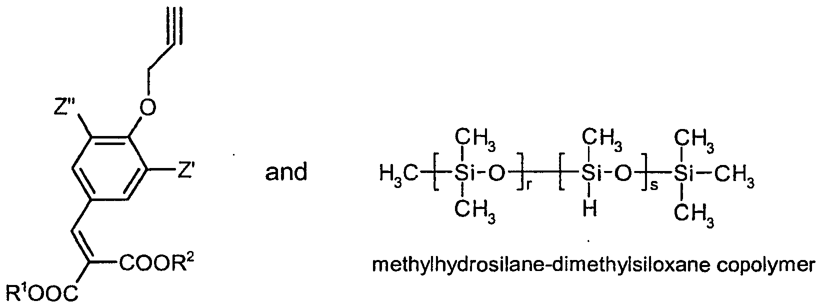 wherein R¹, R², Z' and Z" are as defined above.

The silylation of the 4-(2-propinyloxy)phenyl methylene diethylester may be carried out employing known procedures for the addition of silicon bonded hydrogen atoms to groups containing aliphatic unsaturation. Such reactions are generally catalyzed by a platinum group metal or a complex of such a metal. Examples of catalysts which may be employed are platinum on carbon, chloroplatinic acid, platinum acetyl acetonate, complexes of platinum compounds with unsaturated compounds e.g. olefins and divinyl disiloxanes, complexes of rhodium and palladium compounds and complexes of platinum compounds supported on inorganic substrates. The addition reaction may be performed at reduced, atmospheric or increased pressure. A solvent can be used, e.g. toluene or xylene, in the reaction mixture although the presence of the solvent is not essential. It is also preferred to carry out the reaction at elevated reaction temperatures e.g. from about 50°C to about 150°C.

The process for preparing the coated metal oxide particles of the present invention is not particularly restricted. All types of metal oxide particles which are principally known to be suitable for sunscreen compositions can be used in the process, in particular particles of titanium dioxide, zinc oxide, zirconium oxide, iron oxide, cerium oxide and mixtures of these metal oxides. Furthermore, mixtures of these metal oxides with aluminum oxide and/or silicon dioxide can be used. The origin of the metal oxides is not particularly limited. Metal oxides can be prepared via a pyrogenic process, a sol-gel process, a grinding process, a plasma process, a precipitation process, a hydrothermal process or by a mining process or a combination of the above mentioned processes, to mention only the most common processes for preparing metal oxide particles. Titanium dioxide or Zinc oxide as well as mixtures of these metals oxides with iron oxide, aluminum oxide or with silicon dioxide are preferred.

The metal oxide particles which can be used in the coating process are either commercially available as e.g. pyrogenic titanium dioxide P25 from Degussa or can be prepared by methods known by a skilled person. Chemical mixtures of pyrogenic oxides can be prepared as described e.g. in EP-A 850 876. Examples of titanium silicon mixed oxides and titanium aluminum mixed oxides are disclosed in EP-A 609 533 and examples for silicon aluminum mixed oxides are disclosed e.g. in EP-A 1 084 617. The disclosure of these documents with respect to preferred metal oxide particles to be coated according to the present invention are explicitly included herein by reference.

The crystalline form of the metal oxide may be of any crystal or amorphous type. For example, titanium dioxide may be any type of amorphous, rutil, anastase, brookite or a mixture thereof.

Particularly preferred are the metal oxides titanium dioxide or zinc oxide optionally doped with iron oxide.

The particle size of the particles to be coated according to the process of the present invention in not particularly limited. All particle sizes which are principally useful for incorporating into sunscreen compositions can be coated with the coating composition according to the process of the present invention.

An exemplary process for the coating of the metal oxide particles according to the present invention comprises the dispersion of the micronised metal oxide particles in a suitable solvent, preferably in water so that the dispersion comprises usually 1 to 80 wt.% of the metal oxide particles. To this dispersion an emulsion of the sunscreen on polysiloxane basis in a mixture of water and alcohol or only in alcohol with or without a surface active ingredient such as an emulsifier and/ or a solubilizer is added during agitation if necessary with a high shear homogenizer. Preferably a surface active ingredient is present. During this process the sunscreen on polysiloxane basis is deposited on the highly reactive metal surface. The ratio of the surface active ingredients to the sunscreen on polysiloxane basis is preferably in the range of 1:10 to 10:1. The amount of the emulsion containing the surface active ingredient and the sunscreen on a polysiloxane basis and the concentration of the sunscreen on a polysiloxane basis in the emulsion used in the coating process depends on the desired stabilizing effect. Preferably, the emulsion contains 1 to 50%, more preferably 10 to 40% of the sunscreen on a polysiloxane basis and preferably 1. to 40%, more preferably 10 to 40% of a surface active ingredient.

The ratio (w/w) of the coating with a sunscreen on a polysiloxane basis to the metal oxide particles in the final product is in the range of 1-20%, more preferably of 2-10%.

All percentages and ratios mentioned in this specification are by weight if nothing else is stated or evident.

The reaction product, the coated metal oxide particles, is separated and optionally washed and dried. The separation of the reaction product can be performed by usual processes such as filtration or centrifugation. The washing can be a washing with water or a suitable organic solvent or mixtures of water with organic solvent.

Typically surface active ingredients such as emulsifiers and solubilizers which can be used in the coating process include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof.

Preferred are for example hydrogenated castor oils such as Cremophor RH 40 or 60, Polysorbates such as Tween 20 or Tween 80, Amphisol cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof.

The coated metal oxide particles according to the invention can be dried using known methods such as those disclosed in Ullmann's Encyclopedia of Industrial Chemistry. Other process steps can follow, such as calcinations, grinding procedures, granulation procedures or dispersion of the coated metal oxide particles in suitable liquids.

The primary particle size of the coated metal oxide particles is usually in the range from 2 to 100 nm, preferably in the range of 5 to 50 nm and the secondary particle size is preferably between 0.05 and 50 µm, preferably between 0.1 and 1 µm.

The coated metal oxide particles of the present invention comprise at least one coating which is the polysiloxane sunscreen as defined above.

Other usual coatings can also be present. The other coatings can be applied before, after or together with the coating with a sunscreen on polysiloxane basis. Other coatings which can be used are e.g. listed in EP 1 281 388 and comprise organic coatings such as stearic acid, silicones (silane derivatives such as triethoxycaprylylsilane or siloxane derivatives such as methicone, dimethicone, simethicone) and/or inorganic coatings such as alumina, silica, silicon dioxide and mixtures thereof. The additional coating can be prepared e.g. according to DE 103 33 029, EP-A 1 284 277, US 5 756 788, EP-A 988 853, EP 0988 853, and US 5 562 897.

In a preferred embodiment other usual coatings are absent.

The at least one coating of the metal oxide particles comprises a polysiloxane sunscreen as defined above. Preferably, at least 30%, more preferably at least 50%, of the coating consists of the polysiloxane sunscreen. The rest of the coating can be of any other usual organic or inorganic coating material, usual additives or impurities. More preferably, the coating consists essentially of the polysiloxane sunscreen, which means that the coating consists of the polysiloxane sunscreen but may contain some impurities, by-products or additives. Such impurities, by-products or additives are preferably present in an amount of 10% or less, more preferably in an amount of 5% or less or in an amount of 2% or less, based on the weight of the polysiloxane sunscreen coating.

The metal oxide particles are at least partly covered by the at least one coating which comprises the polysiloxane sunscreen, preferably at least 50% of the surface of the metal oxide particles is covered by this coating, more preferably at least 90%, most preferred the complete particles are covered with this coating.

The coated metal oxide particles of the present invention can be included within cosmetic or dermatological compositions, especially sunscreen compositions in manners well known to a skilled person. The sunscreen compositions are generally topical sunscreen compositions.

For the preparation of the topical sunscreen compositions, especially preparations for dermatological and/or cosmetic use, such as skin protection and sunscreen formulations for everyday cosmetics the coated metal oxide particles of the present invention can be incorporated in auxiliary agents, e.g. a cosmetic base, which are conventionally used for such formulations. Where convenient, other conventional UV-A and/or UV-B and/ or broad spectrum screening agents may also be added. The combination of UV screens may show a synergistic effect. The amount of the coated metal oxide particles of the present invention and other known UV-screens is not critical. Suitable amounts of the coated metal oxide particles of the present invention are about 0.01 to about 50% by weight, preferably about 0.01 to 25 wt.% (depending on the payload and volume fraction of the particles) and about 0.5-12% by weight of at least one additional, hydrophilic and/or lipophilic UV-A or UV-B or broad spectrum screening agent. These additional screening agents are advantageously selected from among the compounds listed below without being limited thereto:

Examples of UV B or broad spectrum screening agents, i.e. substances having absorption maximums between about 290 and 340 nm, which come into consideration for combination with the coated particles of the present invention are for example the following organic and inorganic compounds :
--- Acrylates such as 2-ethylhexyl 2-cyano-3,3-diphenylacrylate (octocrylene, PARSOL^{®} 340), ethyl 2-cyano-3,3-diphenylacrylate and the like;
--- Camphor derivatives such as 4-methyl benzylidene camphor (PARSOL^{®} 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
--- Cinnamate derivatives such as octyl methoxycinnamate (PARSOL^{®} MCX), ethoxyethyl methoxycinnamate, diethanolamine methoxycinnamate (PARSOL^{®} Hydro), isoamyl methoxycinnamate and the like as well as cinnamic acid derivatives bond to siloxanes;
---- p-aminobenzoic acid derivatives, such as p-aminobenzoic acid, 2-ethylhexyl p-dimethylaminobenzoate, N-oxypropylenated ethyl p-aminobenzoate, glyceryl p-aminobenzoate,
--- Benzophenones such as benzophenone-3, benzophenone-4, 2,2', 4, 4'-tetrahydroxy-benzophenone, 2,2'-dihydroxy-4,4'-dimethoxybenzophenone and the like;
--- Esters of benzalmalonic acid such as di-(2-ethylhexyl) 4-methoxybenzalmalonate;
--- Esters of 2-(4-ethoxy-anilinomethylene)propanedioic acid such as 2-(4-ethoxy anilinomethylene)propanedioic acid diethyl ester as described in the European Patent Publication EP 0895 776;
--- Organosiloxane compounds containing benzmalonate groups as described in the European Patent Publications EP 0358584 B1, EP 0538431 B1 and EP 0709080 A1;
--- Drometrizole trisiloxane (Mexoryl XL);
--- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL^{®} HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
--- Triazine derivatives such as octyl triazone (UVINUL T-150), dioctyl butamido triazone (UVASORB HEB), bis ethoxyphenol methoxyphenyl triazine (Tinosorb S) and the like;
--- Encapsulated UV-filters such as encapsulated octyl methoxycinnamate (Eusolex UV-pearls) and the like.

Examples of broad spectrum or UV A screening agents i.e. substances having absorption maximums between about 320 and 400 nm, which come into consideration for combination with the coated particles of the present invention are for example the following organic and inorganic compounds :
--- Dibenzoylmethane derivatives such as 4-tert. butyl-4'-methoxydibenzoylmethane (PARSOL^{®} 1789), dimethoxydibenzoylmethane, isopropyldibenzoylmethane and the like;
--- Benzotriazole derivatives such as 2,2'-methylene-bis-(6-(2H-benzotriazole-2-yl)-4-(1,1,3,3,-tetramethylbutyl)-phenol (TINOSORB M) and the like;
--- phenylene-1,4-bis-benzimidazolsulfonic acids or salts such as 2,2-(1,4-phenylene)bis-(1H-benzimidazol-4,6-disulfonic acid) (Neoheliopan AP);
--- amino substituted hydroxybenzophenones such as 2-(4-Diethylamino-2-hydroxy-benzoyl)-benzoic acid hexylester as described in the European Patent Publication EP 1046391
--- Pigments such as microparticulated ZnO or TiO₂ and the like. The term "microparticulated" refers to a particle size from about 5 nm to about 200 nm, particularly from about 15 nm to about 100 nm. The particles may also be coated by other metal oxides such as e.g. aluminum or zirconium oxides or by organic coatings such as e.g. polyols, methicone, aluminum stearate, alkyl silane. Such coatings are well known in the art.

As dibenzoylmethane derivatives have limited photostability it may be desirable to photostabilize these UV-A screening agents. Thus, the term "conventional UV-A screening agent" also refers to dibenzoylmethane derivatives such as e.g. PARSOL^{®} 1789 stabilized by, e.g.,
--- 3,3-Diphenylacrylate derivatives as described in the European Patent Publications EP 0 514 491 B1 and EP 0 780 119 A1;
--- Benzylidene camphor derivatives as described in the US Patent No. 5,605,680;
--- Organosiloxanes containing benzmalonate groups as described in the European Patent Publications EP 0358584 B1, EP 0538431 B1 and EP 0709080 A1.

The compositions of the invention can also contain usual cosmetic adjuvants and additives, such as preservatives/ antioxidants, fatty substances/ oils, water, organic solvents, silicones, thickeners, softeners; emulsifiers, additional sunscreens, antifoaming agents, moisturizers, fragrances, surfactants, fillers, sequestering agents, anionic, cationic, nonionic or amphoteric polymers or mixtures thereof, propellants, acidifying or basifying agents, dyes, colorants, pigments or nanopigments, in particular those suited for providing an additional photoprotective effect by physically blocking out ultraviolet radiation, or any other ingredients usually formulated into cosmetics, in particular for the production of sunscreen/ antisun compositions. The necessary amounts of the cosmetic and dermatological adjuvants and additives can, based on the desired product, easily be chosen by a skilled artisan in this field and will be illustrated in the examples, without being limited hereto.

An additional amount of antioxidants/ preservatives is generally preferred. Based on the invention all known antioxidants usually formulated into cosmetics can be used. Especially preferred are antioxidants chosen from the group consisting of amino acids (e.g. glycine, histidine, tyrosine, tryptophan) and their derivatives, imidazole (e.g. urocanic acid) and derivatives, peptides such as D,L-carnosine, D-carnosine, L-carnosine and derivatives (e.g. anserine), carotenoids, carotenes (e.g. α-carotene, β-carotene, lycopene) and derivatives, chlorogenic acid and derivatives, lipoic acid and derivatives (e.g. dihydrolipoic acid), aurothioglucose, propylthiouracil and other thiols (e.g. thioredoxine, glutathione, cysteine, cystine, cystamine and its glycosyl-, N-acetyl-, methyl-, ethyl-, propyl-, amyl-, butyl- and lauryl-, palmitoyl-; oleyl-, y-linoleyl-, cholesteryl- and glycerylester) and the salts thereof, dilaurylthiodipropionate, distearylthiodipropionate, thiodipropionic acid and its derivatives (ester, ether, peptides, lipids, nucleotides, nucleosides and salts) as well as sulfoximine compounds (such as buthioninsulfoximine, homocysteinesulfoximine, buthioninsulfone, penta-, hexa-, heptathioninsulfoximine) in very low compatible doses (e.g. pmol bis µmol/kg), additionally (metal)-chelators (such as α-hydroxyfatty acids, palmic-, phytinic acid, lactoferrin), β-hydroxyacids (such as citric acid, lactic acid, malic acid), huminic acid, gallic acid, gallic extracts, bilirubin, biliverdin, EDTA, EGTA and its derivatives, unsaturated fatty acids and their derivatives (such as γ-linoleic acid, linolic acid, oleic acid), folic acid and its derivatives, ubiquinone and ubiquinol and their derivatives, vitamin C and derivatives (such as ascorbylpalmitate and ascorbyltetraisopalmitate, Mg-ascorbylphosphate, Na-ascorbylphosphate, ascorbyl-acetate), tocopherol and derivates (such as vitamin-E-acetate), mixtures of nat. vitamin E, vitamin A and derivatives (vitamin-A-palmitate and -acetate) as well as coniferylbenzoate, rutinic acid and derivatives, α-glycosylrutin, ferulic acid, furfurylideneglucitol, carnosine, butylhydroxytoluene, butylhydroxyanisole, trihydroxybutyrophenone, urea and its derivatives, mannose and derivatives, zinc and derivatives (e.g. ZnO, ZnSO₄), selen and derivatives (e.g. selenomethionin), stilbenes and derivatives (such as stilbenoxide, trans-stilbenoxide) and suitable derivatives (salts, esters, ethers, sugars, nucleotides, nucleosides, peptides and lipids) of the named active ingredients. One or more preservatives/antioxidants may be present in an amount about 0.01 wt.% to about 10 wt.% of the total weight of the composition of the present invention. Preferably, one or more preservatives/antioxidants are present in an amount about 0.1 wt.% to about 1 wt.%.

Typically formulations also contain surface active ingredients like emulsifiers, solubilizers and the like. An emulsifier enables two or more immiscible components to be combined homogeneously. Moreover, the emulsifier acts to stabilize the composition. Emulsifiers that may be used in the present invention in order to form O/W, W/O, O/W/O or W/O/W emulsions/ microemulsions include sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, TEA myristate, TEA stearate, magnesium stearate, sodium stearate, potassium laurate, potassium ricinoleate, sodium cocoate, sodium tallowate, potassium castorate, sodium oleate, and mixtures thereof. Further suitable emulsifiers are phosphate esters and the salts thereof such as cetyl phosphate (Amphisol^{®} A), diethanolamine cetyl phosphate (Amphisol^{®}), potassium cetyl phosphate (Amphisol^{®} K), sodium glyceryl oleate phosphate, hydrogenated vegetable glycerides phosphate and mixtures thereof. Furthermore, one or more synthetic polymers may be used as an emulsifier. For example, PVP eicosene copolymer, acrylates/C₁₀₋₃₀ alkyl acrylate crosspolymer, acrylates/steareth-20 methacrylate copolymer, PEG-22/dodecyl glycol copolymer, PEG-45/dodecyl glycol copolymer, and mixtures thereof. The preferred emulsifiers are cetyl phosphate (Amphisol^{®} A), diethanolamine cetyl phosphate (Amphisol^{®}), potassium cetyl phosphate (Amphisol^{®} K), PVP Eicosene copolymer, acrylates/C₁₀₋₃₀-alkyl acrylate crosspolymer, PEG-20 sorbitan isostearate, sorbitan isostearate, and mixtures thereof. The one or more emulsifiers are present in a total amount about 0.01 wt.% to about 20 wt.% of the total weight of the composition of the present invention. Preferably, about 0.1 wt.% to about 10 wt.% of emulsifiers are used.

The lipid phase can advantageously be chosen from:
mineral oils and mineral waxes;
oils such as triglycerides of caprinic acid or caprylic acid, preferable castor oil;
oils or waxes and other natural or synthetic oils, in an preferred embodiment esters of fatty acids with alcohols e.g. isopropanol, propyleneglycol, glycerin or esters of fatty alcohols with carbonic acids or fatty acids;
alkylbenzoates; and/or
silicone oils such as dimethylpolysiloxane, diethylpolysiloxane, diphenylpolysiloxane, cyclomethicones and mixtures thereof.

Exemplary fatty substances which can be incorporated in the oil phase of the emulsion, microemulsion, oleo gel, hydrodispersion or lipodispersion of the present invention are advantageously chosen from esters of saturated and/or unsaturated, linear or branched alkyl carboxylic acids with 3 to 30 carbon atoms, and saturated and/or unsaturated, linear and/or branched alcohols with 3 to 30 carbon atoms as well as esters of aromatic carboxylic acids and of saturated and/or unsaturated, linear or branched alcohols of 3-30 carbon atoms. Such esters can advantageously be selected from octylpalmitate, octylcocoate, octylisostearate, octyldodecylmyristate, cetearylisononanoate, isopropylmyristate, isopropylpalmitate, isopropylstearate, isopropyloleate, n-butylstearate, n-hexyllaureate, n-decyloleate, isooctylstearate, isononylstearate, isononylisononanoate, 2-ethylhexylpalmitate, 2-ethylhexyllaurate, 2-hexyldecylstearate, 2-octyldodecylpalmitate, stearylheptanoate, oleyloleate, oleylerucate, erucyloleate, erucylerucate, tridecylstearate, tridecyltrimellitate, as well as synthetic, half-synthetic or natural mixtures of such esters e.g. jojoba oil.

Other fatty components suitable for use in the formulation of the present invention include polar oils such as lecithins and fatty acid triglycerides, namely triglycerol esters of saturated and/or unsaturated, straight or branched carboxylic acid with 8 to 24 carbon atoms, preferably of 12 to 18 carbon-atoms whereas the fatty acid triglycerides are preferably chosen from synthetic, half synthetic or natural oils (e.g. cocoglyceride, olive oil, sun flower oil, soybean oil, peanut oil, rape seed oil, sweet almond oil, palm oil, coconut oil, castor oil, hydrogenated castor oil, wheat oil, grape seed oil, macadamia nut oil and others); apolar oils such as linear and/ or branched hydrocarbons and waxes e.g. mineral oils, vaseline (petrolatum); paraffins, squalane and squalene, polyolefins, hydrogenated polyisobutenes and isohexadecanes, favored polyolefins are polydecenes; dialkyl ethers such as dicaprylylether; linear or cyclic silicone oils such as preferably cyclomethicone (octamethylcyclotetrasiloxane; cetyldimethicone, hexamethylcyclotrisiloxane, polydimethylsiloxane, poly(methylphenylsiloxane) and mixtures thereof.

Other fatty components which can advantageously be incorporated in formulations of the present invention are isoeikosane; neopentylglycoldiheptanoate; propyleneglycoldicaprylate/ dicaprate; caprylic/ capric/ diglycerylsuccinate; butyleneglycol caprylat/caprat; C₁₂-₁₃-alkyllactate; di-C₁₂-₁₃-alkyltartrate; triisostearin; dipentaerythrityl hexacaprylat/hexacaprate; propyleneglycolmonoisostearate; tricaprylin; dimethylisosorbid. Especially beneficial is the use of mixtures C₁₂₋₁₅-alkylbenzoate and 2-ethylhexylisostearate, mixtures C₁₂₋₁₅-alkylbenzoate and isotridecylisononanoate as well as mixtures of C₁₂₋₁₅-alkylbenzoate, 2-ethylhexylisostearate and isotridecylisononanoate.

The oily phase of the formulation of the present invention can also contain natural vegetable or animal waxes such as bee wax, china wax, bumblebee wax and other waxes of insects as well as shea butter and cocoa butter.

A moisturizing agent may be incorporated into a composition of the present invention to maintain hydration or rehydrate the skin. Moisturizers that prevent water from evaporating from the skin by providing a protective coating are called emollients. Additionally an emollient provides a softening or soothing effect on the skin surface and is generally considered safe for topical use. Preferred emollients include mineral oils, lanolin, petrolatum, capric/caprylic triglyceraldehydes, cholesterol, silicones such as dimeticone, cyclometicone, almond oil, jojoba oil, avocado oil, castor oil, sesame oil, sunflower oil, coconut oil and grape seed oil, cocoa butter, olive oil aloe extracts, fatty acids such as oleic and stearic, fatty alcohols such as cetyl and hexadecyl (ENJAY), diisopropyl adipate, hydroxybenzoate esters, benzoic acid esters of C₉₋₁₅-alcohols, isononyl iso-nonanoate, ethers such as polyoxypropylene butyl ethers and polyoxypropylene cetyl ethers, and C₁₂₋₁₅-alkyl benzoates, and mixtures thereof. The most preferred emollients are hydroxybenzoate esters, aloe vera, C₁₂₋₁₅-alkyl benzoates, and mixtures thereof. An emollient is present in an amount of about 1 wt.% to about 20 wt.% of the total weight of the composition. The preferred amount of emollient is about 2 wt.% to about 15 wt.%, and most preferably about 4 wt.% to about 10 wt.%.

Moisturizers that bind water, thereby retaining it on the skin surface are called humectants. Suitable humectants can be incorporated into a composition of the present invention such as glycerin, polypropylene glycol, polyethylene glycol, lactic acid, pyrrolidone carboxylic acid, urea, phospholipids, collagen, elastin, ceramides, lecithin sorbitol, PEG-4, and mixtures thereof. Additional suitable moisturizers are polymeric moisturizers of the family of water soluble and/ or swellable/ and/ or with water gelating polysaccharides such as hyaluronic acid, chitosan and/or a fucose rich polysaccharide which is e.g. available as Fucogel^{®} 1000 (CAS-Nr. 178463-23-5) by SOLABIA S. One or more humectants are optionally present at about 0.5 wt.% to about 8 wt.% in a composition of the present invention, preferably about 1 wt.% to about 5 wt.%.

The aqueous phase of the compositions of the present invention can contain the usual cosmetic additives such as alcohols, especially lower alcohols, preferably ethanol and/ or isopropanol, low diols or polyols and their ethers, preferably propyleneglycol, glycerin, ethyleneglycol, ethyleneglycol monoethyl- or monobutylether, propyleneglycol monomethyl-or -monoethyl- or-monobutylether, diethyleneglycol monomethyl-or monoethylether and analogue products, polymers, foam stabilizers; electrolytes and especially one or more thickeners. Thickeners that may be used in formulations of the present invention to assist in making the consistency of a product suitable include carbomer, silicon dioxide, magnesium and/ or aluminum silicates, beeswax, stearic acid, stearyl alcohol polysaccharides and their derivatives such as xanthan gum, hydroxypropyl cellulose, polyacrylamides, acrylate crosspolymers preferably a carbomer, such as carbopole^{®} of type 980, 981, 1382, 2984, 5984 alone or mixtures thereof. Suitable neutralizing agents which may be included in the composition of the present invention to neutralize components such as e.g. an emulsifier or a foam builder/stabilizer include but are not limited to alkali hydroxides such as a sodium and potassium hydroxide; organic bases such as diethanolamine (DEA), triethanolamine (TEA), aminomethyl propanol, and mixtures thereof; amino acids such as arginine and lysine and any combination of any foregoing. The neutralizing agent can be present in an amount of about 0.01 wt.% to about 8 wt.% in the composition of the present invention, preferably, 1 wt.% to about 5 wt.%.

The addition of electrolytes into the composition of the present invention may be necessary to change the behavior of a hydrophobic emulsifier. Thus, the emulsions/ microemulsions of this invention may contain preferably electrolytes of one or several salts including anions such as chloride, sulfates, carbonate, borate and aluminate, without being limited thereto. Other suitable electrolytes can be on the basis of organic anions such as, but not limited to, lactate, acetate, benzoate, propionate, tartrate and citrate. As cations preferably ammonium, alkylammonium, alkali- or alkaline earth metals, magnesium-, iron- or zinc-ions are selected. Especially preferred salts are potassium and sodium chloride, magnesium sulfate, zinc sulfate and mixtures thereof. Electrolytes can be present in an amount of about 0.01 wt.% to about 8 wt.% in the composition of the present invention.

The cosmetic compositions of the invention are useful as compositions for photoprotecting the human epidermis or hair against the damaging effect of ultraviolet irradiation, as sunscreen compositions. Such compositions can, in particular, be provided in the form of a lotion, a thickened lotion, a gel, a cream, a milk, an ointment, a powder or a solid tube stick and can be optionally be packaged as an aerosol and can be provided in the form of a mousse, foam or a spray. When the cosmetic composition according to the invention are provided for protecting the human epidermis against UV radiation or as sunscreen composition, they can be in the form of a suspension or dispersion in solvents or fatty substances, or alternatively in the form of an emulsion or microemulsion (in particular of O/W or W/O type, O/W/O or W/O/W-type), such as a scream or a milk, a vesicular dispersion, in the form of an ointment, a gel, a solid tube stick or an aerosol mousse. The emulsions can also contain anionic, nonionic, cationic or amphoteric surfactants.

The following examples are provided to further illustrate the processes and compositions of the present invention. These examples are illustrative only and are not intended to limit the scope of the invention in any way.

### Preparation of silica-coated titanium dioxide

The coating of the metal oxide coated with a sunscreen on polysiloxane basis was measured by FT-IR spectrometry using a Perkin Elmer Spectrum One, FT-IR Spectrometer and on determination of the resulting transmission infrared absorption spectrum: a peak originating from the Si-O-Si stretching vibration was observed from 1000 to 1200 cm⁻¹. The incorporation of the sunscreen on polysiloxane basis is proven by a peak originating from the carbonyl group of the chromophore at about 1650-1720cm⁻¹. The effectiveness of the coating is proven via the method described below showing a reduced coloration of all samples prepared according to the invention compared to uncoated titanium dioxide.

### Stability during irradiation

Untreated titanium dioxide produces an intense yellow coloration upon irradiation with UV-light. The more intense the color, the greater the reactivity of the titanium dioxide. This offers a good analytical test for the effectiveness of the surface treatment.

A 10% dispersion of the coated TiO₂ in Caprylic/Capric Triglyceride in comparison to an uncoated sample and also in comparison to Uvinul TiO₂. is drawn as a film on a glass plate with a 20 µm spreading knife. Afterwards irradiation was performed with a Hereaus Suntester with 40 MED. The judgment of the color of the samples was performed by comparison with "Methuen Handbook of Color", A. Kornerup und J.H. Wanscher, 3. edition, Eyre Methuen, London, 1984. Additionally, the samples were compared with untreated TiO₂ and with a commercially available silicone coated titanium dioxide grade: Uvinul^{®} TiO₂ (octylsilylated titanium dioxide from BASF).

The surface treated sample of this invention showed a reduced coloration compared to the untreated TiO₂ and an equal or better performance compared to the commercial sample, Uvinul^{®} TiO₂ (octylsilylated titanium dioxide).

| TiO₂ | Color* after irradiation with 40 MED |
|---|---|
| Non-treated TiO₂ | pastel yellow |
| Uvinul^{®} TiO₂ (octylsilylated titanium dioxide) | pale yellow |
| Example 1 | white |

| | |
|---|---|
| *according to Table 3 "Methuen Handbook of Color", A. Kornerup und J.H. Wanscher, 3. edition. | |

### Example 1

Into a dispersion of 40 g micronized titanium dioxide in 60 ml water, 4 g of a (w/w) mixture of 30% polysilicone 15, 30% PEG-40 hydrogenated castor oil and 20% water and 20% ethanol is added and homogenized by using a Ultra Turrax T 25 (IKA) at room temperature for 15 minutes. After that the dispersion was centrifuged and the aqueous phase was removed. The preparation was washed using a mixture of 30% ethanol in water to remove the surplus of PEG-40 hydrogenated castor oil. The product was dried at 120°C and milled to guaranty a fine primary particle size.

Determination of the in vitro SPF of an O/W emulsion as described e.g. in example 2 containing as UV filter different concentrations of TiO₂ coated with polysilicone-15 as described in example 1 and 2% of PARSOL^{®} 1789.

| Formula | In vitro SPF* |
|---|---|
| 4% TiO₂ as described in example 1 | 17 |
| 6% TiO₂ as described in example 1 | 30 |
| 8% TiO₂ as described in example 1 | 38 |

| | |
|---|---|
| *Measured with Optometrics SPF 290 | |

### Example 2

### O/W sun milk

| | Ingredients | INCI Nomenclature | % w / w |
|---|---|---|---|
| A) | PARSOL SLX^{®} | Polysilicone-15 | 6.00 |
| | Neo Heliopan AP | | 3.00 |
| | Tinosorb S | Hydrogenated Cocoglycerides | 3.00 |
| | Lanette O | Cetearyl Alcohol | 2.00 |
| | Myritol 318 | Caprylic/capric Triglyceride | 6.00 |
| | Mineral oil | Mineral oil | 2.00 |
| | Vitamin E acetate | Tocopheryl Acetate | 1.00 |
| | Prisorine 3515 | Isostearyl Alcohol | 4.00 |
| B) | Edeta BD | Disodium EDTA | 0.10 |
| | Phenonip | Phenoxyethanol & Methylparaben & Ethylparaben & Propylparaben & Butylparaben | 0.60 |
| | Amphisol^{®} K | Potassium Cetyl Phosphate | 2.00 |
| | Water deionized | Aqua | ad100 |
| | 1,2-Propylen Glycol | Propylene Glycol | 5.00 |
| | Carbopol 981 | Carbomer | 0.30 |
| | Tinosorb M | Methylene Bis-Benzotriazolyl Tetramethylbutylphenol | 6.00 |
| | KOH 10% solution | Potassium Hydroxyde | 2.10 |
| C) | Polysiloxane coated metal oxide | | 0.01-25 |

### Procedure:

Heat part A) and B) to 85°C while stirring. When homogeneous, add part B) to A) under agitation. Cool to ambient temperature while stirring and add part C). Homogenize to achieve a small particle size.

### Example 3

Sun milk waterproofed

| | Ingredients | INCI Nomenclature | % w / w |
|---|---|---|---|
| A) | PARSOL SLX^{®} | Polysilicone-15 | 6.00 |
| | PARSOL^{®} 1789 | Butyl Methoxydibenzoylmethane | 2.00 |
| | PARSOL^{®} 5000 | 4-Methylbenzylidene Camphor | 4.00 |
| | Uvinul T 150 | Ethylhexyltriazone | 2.00 |
| | Silicone DC 200/350 cs | Dimethicone | 1.00 |
| | Lanette O | Cetearyl Alcohol | 2.00 |
| | Softisan 100 | Hydrogenated Coco-Glycerides | 3.00 |
| | Tegosoft TN | C12-15 Alkyl Benzoate | 6.00 |
| | Cetiol B | Dibutyl Adipate | 7.00 |
| | Vitamin E acetate | Tocopheryl Acetate | 2.00 |
| | BHT | BHT | 0.05 |
| | Edeta BD | Disodium EDTA | 0.10 |
| | Phenonip | Phenoxyethanol & Methylparaben & Ethylparaben & Propylparaben & Butylparaben | 0.60 |
| | Amphisol^{®} | Cetyl Phosphate DEA | 2.00 |
| B) | Water deionized | Aqua | ad 100 |
| | Propylene Glycol | Propylene Glycol | 5.00 |
| | Carbopol 980 | Carbomer | 0.30 |
| | KOH(10% sol.) | Potassium Hydroxide | 1.50 |
| C) | Polysiloxane coated metal oxide | | 0.01-25 |

### Procedure:

Heat part A) and B) to 85°C while stirring. When homogeneous, add part B) to A) under agitation. Cool to ambient temperature while stirring and add part C). Homogenize to achieve a small particle size.

### Example 4

### Sun milk for babies and children

| | Ingredients | INCI Nomenclature | % w / w |
|---|---|---|---|
| A) | Tegosoft TN | C12-15 Alkyl Benzoate | 5.00 |
| | Silicone 2503 Cosmetic Wax | Stearyl Dimethicone | 2.00 |
| | Cetyl Alcohol | Cetyl Alcohol | 1.00 |
| | Butylated Hydroxytoluene | BHT | 0.05 |
| | Estol GMM 3650 | Glyceryl Myristate | 4.00 |
| | Edeta BD | Disodium EDTA | 0.10 |
| | Phenonip | Phenoxyethanol & Methylparaben & Ethylparaben & Propylparaben & Butylparaben | 0.60 |
| | Amphisol^{®} A | Cetyl Phosphate | 2.00 |
| B) | Water deionized | Aqua | ad 100 |
| | Carbopol 980 | Carbomer | 0.6 |
| | Glycerin | Glycerin | 3.00 |
| | KOH sol. 10% | Potassium Hydroxide | 2.4 |
| C) | Polysiloxane coated metal oxide | | 0.01-25 |

### Procedure:

Heat part A) and B) to 85°C while stirring. When homogeneous, add part B) to A) under agitation. Cool to ambient temperature while stirring and add part C). Homogenize to achieve a small particle size.

### Example 5

High protective sun milk

| | Ingredients | INCI Nomenclature | % w / w |
|---|---|---|---|
| A) | PARSOL SLX^{®} | Polysilicone-15 | 6.00 |
| | PARSOL^{®} 1789 | Butyl Methoxydibenzoylmethane | 2.00 |
| | PARSOL^{®} 5000 | 4-Methylbenzylidene Camphor | 4.00 |
| | Uvinul^{®} T 150 | | 2.00 |
| | Silicone DC 200/350 cs | Dimethicone | 1.00 |
| | Lanette O | Cetearyl Alcohol | 2.00 |
| | Softisan 100 | Hydrogenated Coco-Glycerides | 3.00 |
| | Tegosoft TN | C12-15 Alkyl Benzoate | 6.00 |
| | Cetiol B | Dibutyl Adipate | 7.00 |
| | Vitamin E acetate | Tocopheryl Acetate | 2.00 |
| | BHT | BHT | 0.05 |
| | Edeta BD | Disodium EDTA | 0.10 |
| | Phenonip | Phenoxyethanol & Methylparaben & Ethylparaben & Propylparaben & Butylparaben | 0.60 |
| | Amphisol^{®} K | Potassium Cetyl Phosphate | 2.00 |
| B) | Water deionized | Aqua | ad 100 |
| | Propylene Glycol | Propylene Glycol | 5.00 |
| | Carbopol 980 | Carbomer | 0.30 |
| | KOH(10%sol.) | Potassium Hydroxide | 1.50 |
| C) | Polysiloxane coated metal oxide | | 0.01-25 |
| D) | Perfume | Perfume | q.s. |

### Procedure:

Heat part A) and B) to 85°C while stirring. When homogeneous, add part B) to A) under agitation. Cool to ambient temperature while stirring and add part C) and D). Homogenize to achieve a small particle size.

### Example 6

Water-free sun gel

| | Ingredients | INCI Nomenclature | % w / w |
|---|---|---|---|
| A) | PARSOL^{®} MCX | Ethylhexyl Methoxycinnamate | 6.00 |
| | PARSOL^{®} 1789 | Butyl Methoxydibenzoylmethane | 4.00 |
| | PARSOL^{®} 5000 | 4-Methylbenzylidene Camphor | 4.00 |
| | Uvasorb HEB | Diethylhexyl Butamido Triazone | 1.50 |
| | Uvinul^{®} A plus | | 2.00 |
| | Vitamin E acetate | Tocopheryl Acetate | 1.50 |
| | Tegosoft TN | C12-15 Alkyl Benzoate | 9.00 |
| | Elefac I-205 | Ethylhexyldodecyl Neopentanoate | 2.00 |
| | Alcohol | Alcohol | ad 100 |
| | Isopropyl Alcohol | Isopropyl Alcohol | 20.00 |
| B) | Klucel MF | Hydroxypropylcellulose | 2.00 |
| C) | Polysiloxane coated metal oxide | | 0.01-25 |
| D) | Perfume | | q.s. |

### Procedure:

Mix part A) and B) while stirring. When homogeneous, add part C) and D) under agitation.

### Example 7

### Sun gel

| | Ingredients | INCI Nomenclature | % w / w |
|---|---|---|---|
| A) | Pemulen TR-2 | Acrylates/C10-30 Alky Acrylate Crosspolymer | 0.60 |
| | Phenonip | Phenoxyethanol & Methylparaben & Ethylparaben & Propylparaben & Butylparaben | 0.60 |
| | Edeta BD | Disodium EDTA | 0.1 |
| | Aqua | Aqua | ad 100 |
| B) | PARSOL^{®} 1789 | Butyl Methoxydibenzoylmethane | 4.00 |
| | PARSOL^{®} 340 | Octocrylene | 3.00 |
| | Tegosoft TN | C12-15 Alkyl Benzoate | 15.00 |
| | Antaron V-216 | PVP/Hexadecene Copolymer | 1.00 |
| | Vitamin E acetate | Tocopheryl Acetate | 0.50 |
| | Butylated Hydroxytoluene | BHT | 0.05 |
| | Cremophor RH 410 | PEG-40 Hydrogenated Castor Oil | 0.50 |
| | Tris Amino | Tromethamine | 0.50 |
| C) | Polysiloxane coated metal oxide | | 0.01-25 |
| D) | Perfume | Perfume | q.s. |

### Procedure:

Heat part A) and B) to 85°C while stirring. When homogeneous, add part B) to A) under agitation. Cool to ambient temperature while stirring and add part C) and D). Homogenize to achieve a small particle size.

### Example 8

### High protection W/O sun milk

| | Ingredients | INCI Nomenclature | % w / w |
|---|---|---|---|
| A) | PARSOL^{®} 1789 | Butyl Methoxydibenzoylmethane | 2.00 |
| | PARSOL^{®} 5000 | 4-Methylbenzylidene Camphor | 4.00 |
| | Uvinul^{®} T 150 | Ethylhexyl Triazone | 2.00 |
| | Uvinul^{®} TiO2 | Titanium Dioxide and Trimethoxycaprylylsilane | 5.00 |
| | Arlacel P 135 | PEG-30 Dipolyhydroxystearate | 2.00 |
| | Tegosoft TN | C12-15 Alkyl Benzoate | 5.00 |
| | Cosmacol EMI | Di-C12-13 Alkyl Malate | 6.00 |
| | Miglyol 840 | Propylene Glycol Dicaprylate/Dicaprate | 6.00 |
| | Butylated Hydroxytoluene | BHT | 0.05 |
| | Phenonip | Phenoxyethanol & Methylparaben & Ethylparaben & Propylparaben & Butylparaben | 0.60 |
| B) | Deionized water | Aqua | ad 100 |
| | Glycerin | Glycerin | 5.00 |
| | Edeta | Disodium EDTA | 0.1 |
| | NaCl | Sodium Chloride | 0.30 |
| C) | PARSOL^{®} HS | Phenylbenzyimidazole Sulphonic Acid | 4.00 |
| | Water | Aqua | 20.00 |
| | Triethanolamine 99%. | Triethanolamine | 2.50 |
| D) | Polysiloxane coated metal oxide | | 0.01-25 |
| E) | Perfume | | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

### Example 9

### W/O milk with pigments

| | Ingredients | INCI Nomenclature | % w / w |
|---|---|---|---|
| A) | Cremophor WO 7 | PEG-7 Hydrogenated Castor Oil | 6.00 |
| | Elfacos ST 9 | PEG-45/Dodecyl Glycol Copolymer | 2.00 |
| | PARSOL^{®} 1789 | Butyl Methoxydibenzoylmethane | 3.00 |
| | Tinosorb S | | 5.00 |
| | PARSOL^{®} 5000 | 4-Methylbenzylidene Camphor | 4.00 |
| | microfine ZnO | Zinc Oxide | 2.00 |
| | Microcrystalline wax | Microcrystalline Wax | 2.00 |
| | Miglyol 812 | Caprylic/capric Triglyceride | 5.00 |
| | Vitamin E acetate | Tocopheryl Acetate | 1.00 |
| | Jojoba oil | Simmondsia Chinensis Seed Oil | 5.00 |
| | Edeta BD | Disodium EDTA | 0.10 |
| | Butylated Hydroxytoluene | BHT | 0.05 |
| | Phenonip | Phenoxyethanol & Methylparaben & Ethylparaben & Propylparaben & Butylparaben | 0.60 |
| B) | Water deionized | Aqua | ad 100 |
| | Glycerin | Glycerin | 5.00 |
| C) | Neo Heliopan AP | | 2.00 |
| | Water deionized | Aqua | 20.00 |
| | KOH 10% solution | Potassium Hydroxide | 4.00 |
| D) | Polysiloxane coated metal oxide | | 0.01-25 |
| E) | Perfume | Perfume | q.s. |

### Procedure:

Heat part A), B) and C) to 85°C while stirring. When homogeneous, add part B) and C) to A) under agitation. Cool to ambient temperature while stirring and add part D) and E). Homogenize to achieve a small particle size.

### Example 10

Protective Day cream with Vitamin C

| | ingredients | INCI Nomenclature | % w / w |
|---|---|---|---|
| A) | PARSOL SLX^{®} | Polysilicone-15. | 4.00 |
| | PARSOL^{®} 1789 | Butyl Methoxydibenzoylmethane | 1.50 |
| | Glyceryl Myristate | Glyceryl Myristate | 2.00 |
| | Cetyl Alcohol | Cetyl Alcohol | 0.50 |
| | Myritol 318 | Caprylic/Capric Triglyceride | 5.00 |
| | Crodamol DA | Diisopropyl Adipate | 5.00 |
| | Vitamin E acetate | Tocopheryl Acetate | 2.00 |
| | Butylated Hydroxytoluene | BHT | 0.05 |
| | Phenonip | Phenoxyethanol & Methylparaben & Ethylparaben & Propylparaben & Butylparaben | 0.60 |
| | Edeta BD | Disodium EDTA | 0.10 |
| | Amphisol^{®} K | Potassium Cetyl Phosphate | 2.00 |
| B) | Water deionized | Aqua | ad 100 |
| | 1,2-Propylene Glycol | Propylene Glycol | 2.00 |
| | D-Panthenol 75 L | Panthenol | 2.00 |
| | Ethanol | Ethanol | 5.00 |
| | Allantoin | Allantoin | 0.20 |
| | Carbopol ETD 2001 | Carbomer | 0.30 |
| | KOH 10% sol. | Potassium Hydroxide | 1.50 |
| C) | Water | Aqua | 10.00 |
| | Stay-C 50 | Sodium Ascorbyl Phosphate | 0.50 |
| D) | Polysiloxane coated metal oxide | | 0.01-25 |
| E) | Perfume | Perfume | q.s. |

## Claims

1. Coated metal oxide particles comprising metal oxide particles and at least one coating thereon, wherein the at least one coating comprises a sunscreen on polysiloxane basis.

2. Coated metal oxide particles according to claim 1, wherein the metal oxide is selected from the group of titanium dioxide, zinc oxide, zircon oxide, iron oxide, cerium oxide, mixtures of the above and mixtures of the above with aluminum oxide, and/or silicon dioxide.

3. Coated metal oxide particles according to claim 2, wherein the metal oxide is titanium dioxide or zinc oxide or iron oxide or a mixture of the above or a mixture of the above with aluminum oxide, and/or silicon dioxide.

4. Coated metal oxide particles according to claim 3, wherein the metal oxide is titanium dioxide optionally doped with iron oxide.

5. Coated metal oxide particles according to any of claims 1 to 4, wherein the sunscreen on polysiloxane basis is polysilicone-15.

6. Coated metal oxide particles according to any of claims 1 to 5, wherein the ratio of the at least one coating comprising a sunscreen on a polysiloxane basis to the metal oxide particles is 1-20% by weight.

7. Coated metal oxide particles according to claim 6, wherein the ratio of the at least one coating with a sunscreen on a polysiloxane basis to the metal oxide particles is 2-10% by weight.

8. Coated-metal oxide particles according to any of claims 1 to 7, wherein the primary particle size of the coated oxide particles is in the range from 2 to 100 nm.

9. Coated metal oxide particles according to any of claims 1 to 8, wherein the at least one coating which comprises the sunscreen on polysiloxane basis completely covers the surface of the metal particles.

10. Coated metal oxide particles according to any of claims 1 to 9, wherein the at least one coating which comprises the sunscreen on polysiloxane basis consists essentially of the sunscreen on polysiloxane basis.

11. Coated metal oxide particles according to claim 10, wherein the metal oxide particles are coated by one coating.

12. Process for producing coated metal oxide particles according to claim 1 to 11, wherein metal oxide particles are contacted as a dispersion with the sunscreen on polysiloxane basis.

13. Process for producing coated metal oxide particles according to claim 12 wherein the metal oxide particles are coated by contacting them in the form of an aqueous dispersion in the presence of a surface active ingredient with the sunscreen on polysiloxane basis.

14. Cosmetic or dermatological composition comprising the coated metal oxide particles as defined in any of claims 1-11.

15. Cosmetic or dermatological composition according to claim 14 wherein additionally further UV-A screening agent/s and/or UV-B screening agent/s and/ or broadband screening agent/s are present.

16. Cosmetic or dermatological composition according to any of claims 14 to15 comprising from 0.01 % - 25 % by weight of the coated metal oxide particles.

17. Use of the coated metal oxide particles as defined in any of claims 1-11 for the stabilization of UV-filters.

18. Use according to claim 17 for the stabilization of dibenzoyl methane or a derivative thereof.

19. The coated metal oxide particles as defined in any of claims 1 to 11 for use for the protection of human skin and/or hair against UV-radiation.

## Patentansprüche

1. Beschichtete Metalloxidteilchen, umfassend Metalloxidteilchen und mindestens eine Beschichtung darauf, wobei die mindestens eine Beschichtung ein Sonnenschutzmittel auf Polysiloxanbasis umfasst.

2. Beschichtete Metalloxidteilchen nach Anspruch 1, wobei das Metalloxid aus der Gruppe von Titandioxid, Zinkoxid, Zirconiumdioxid, Eisenoxid, Ceroxid, Gemischen der obigen und Gemischen der obigen mit Aluminiumoxid und/oder Siliciumdioxid ausgewählt sind.

3. Beschichtete Metalloxidteilchen nach Anspruch 2, wobei das Metalloxid Titandioxid oder Zinkoxid oder Eisenoxid oder ein Gemisch der obigen oder ein Gemisch der obigen mit Aluminiumoxid und/oder Siliciumdioxid ist.

4. Beschichtete Metalloxidteilchen nach Anspruch 3, wobei das Metalloxid Titandioxid, gegebenenfalls dotiert mit Eisenoxid, ist.

5. Beschichtete Metalloxidteilchen nach einem der Ansprüche 1 bis 4, wobei das Sonnenschutzmittel auf Polysiloxanbasis Polysilikon-15 ist.

6. Beschichtete Metalloxidteilchen nach einem der Ansprüche 1 bis 5, wobei das Verhältnis der mindestens einen Beschichtung, umfassend ein Sonnenschutzmittel auf Polysiloxanbasis, zu den Metalloxidteilchen 1 - 20 Gew.-% beträgt.

7. Beschichtete Metalloxidteilchen nach Anspruch 6, wobei das Verhältnis der mindestens einen Beschichtung mit einem Sonnenschutzmittel auf Polysiloxanbasis zu den Metalloxidteilchen 2 - 10 Gew.-% beträgt.

8. Beschichtete Metalloxidteilchen nach einem der Ansprüche 1 bis 7, wobei die primäre Teilchengröße der beschichteten Oxidteilchen im Bereich von 2 bis 100 nm liegt.

9. Beschichtete Metalloxidteilchen nach einem der Ansprüche 1 bis 8, wobei die mindestens eine Beschichtung, die das Sonnenschutzmittel auf Polysiloxanbasis umfasst, die Oberfläche der Metallteilchen vollständig bedeckt.

10. Beschichtete Metalloxidteilchen nach einem der Ansprüche 1 bis 9, wobei die mindestens eine Beschichtung, die das Sonnenschutzmittel auf Polysiloxanbasis umfasst, im Wesentlichen aus dem Sonnenschutzmittel auf Polysiloxanbasis besteht.

11. Beschichtete Metalloxidteilchen nach Anspruch 10, wobei die Metalloxidteilchen mit einer Beschichtung beschichtet sind.

12. Verfahren zur Herstellung beschichteter Metalloxidteilchen nach Anspruch 1 bis 11, wobei die Metalloxidteilchen als eine Dispersion mit dem Sonnenschutzmittel auf Polysiloxanbasis kontaktiert werden.

13. Verfahren zur Herstellung beschichteter Metalloxidteilchen nach Anspruch 12, wobei die Metalloxidteilchen durch Kontaktieren derselben in Form einer wässerigen Dispersion in Gegenwart eines oberflächenaktiven Wirkstoffs mit dem Sonnenschutzmittel auf Polysiloxanbasis beschichtet werden.

14. Kosmetische oder dermatologische Zusammensetzung, umfassend die beschichteten Metalloxidteilchen, wie in einem der Ansprüche 1 - 11 definiert.

15. Kosmetische oder dermatologische Zusammensetzung nach Anspruch 14, wobei überdies ferner UV-A-Schutzmittel und/oder UV-B-Schutzmittel und/oder Breitbandschutzmittel vorliegen.

16. Kosmetische oder dermatologische Zusammensetzung nach einem der Ansprüche 14 bis 15, umfassend 0,01 Gew.-% - 25 Gew.-% der beschichteten Metalloxidteilchen.

17. Verwendung der beschichteten Metalloxidteilchen, wie in einem der Ansprüche 1-11 definiert, zur Stabilisierung von UV-Filtern.

18. Verwendung nach Anspruch 17 zur Stabilisierung von Dibenzoylmethan oder einem Derivat davon.

19. Beschichtete Metalloxidteilchen, wie in einem der Ansprüche 1 bis 11 definiert, zur Verwendung als Schutz von menschlicher Haut und/oder Haar gegen UV-Strahlung.

## Revendications

1. Particules d'oxyde métallique revêtues comprenant des particules d'oxyde métallique sur lesquelles au moins un revêtement est appliqué, dans lesquelles le au moins un revêtement comprend une protection solaire à base de polysiloxane.

2. Particules d'oxyde métallique revêtues selon la revendication 1, dans lesquelles l'oxyde métallique est sélectionné dans le groupe constitué du dioxyde de titane, de l'oxyde de zinc, de l'oxyde de zirconium, de l'oxyde de fer, de l'oxyde de cérium, de mélanges des composés précités et de mélanges des composés précités avec de l'oxyde d'aluminium et/ou du dioxyde de silicium.

3. Particules d'oxyde métallique revêtues selon la revendication 2, dans lesquelles l'oxyde métallique est le dioxyde de titane ou l'oxyde de zinc ou l'oxyde de fer ou un mélange des composés précités ou un mélange des composés précités avec de l'oxyde d'aluminium et/ou du dioxyde de silicium.

4. Particules d'oxyde métallique revêtues selon la revendication 3, dans lesquelles l'oxyde métallique est le dioxyde de titane éventuellement dopé avec de l'oxyde de fer.

5. Particules d'oxyde métallique revêtues selon l'une quelconque des revendications 1 à 4, dans lesquelles la protection solaire à base de polysiloxane est une polysilicone-15.

6. Particules d'oxyde métallique revêtues selon l'une quelconque des revendications 1 à 5, dans lesquelles le rapport du au moins un revêtement comprenant une protection solaire à base de polysiloxane aux particules d'oxyde métallique est de 1 à 20 % en poids.

7. Particules d'oxyde métallique revêtues selon la revendication 6, dans lesquelles le rapport du au moins un revêtement avec une protection solaire à base de polysiloxane aux particules d'oxyde métallique est de 2 à 10 % en poids.

8. Particules d'oxyde métallique revêtues selon l'une quelconque des revendications 1 à 7, dans lesquelles la taille particulaire primaire des particules d'oxyde revêtues se situe dans la plage de 2 à 100 nm.

9. Particules d'oxyde métallique revêtues selon l'une quelconque des revendications 1 à 8, dans lesquelles le au moins un revêtement qui comprend la protection solaire à base de polysiloxane recouvre complètement la surface des particules métalliques.

10. Particules d'oxyde métallique revêtues selon l'une quelconque des revendications 1 à 9, dans lesquelles le au moins un revêtement qui comprend la protection solaire à base de polysiloxane est essentiellement constitué de la protection solaire à base de polysiloxane.

11. Particules d'oxyde métallique revêtues selon la revendication 10, dans lesquelles les particules d'oxyde métallique sont recouvertes d'un seul revêtement.

12. Procédé pour produire des particules d'oxyde métallique revêtues selon les revendications 1 à 11, dans lequel les particules d'oxyde métallique sont mises en contact, sous la forme d'une dispersion, avec la protection solaire à base de polysiloxane.

13. Procédé pour produire les particules d'oxyde métallique revêtues selon la revendication 12, dans lequel les particules d'oxyde métallique sont revêtues en les mettant en contact, sous la forme d'une dispersion aqueuse en présence d'un ingrédient tensioactif, avec la protection solaire à base de polysiloxane.

14. Composition cosmétique ou dermatologique comprenant les particules d'oxyde métallique revêtues selon l'une quelconque des revendications 1 à 11.

15. Composition cosmétique ou dermatologique selon la revendication 14, dans laquelle sont également présents en supplément un ou plusieurs agents de protection contre les UV-A et/ou un ou plusieurs agents de protection contre les UV-B et/ou un ou plusieurs agents de protection contre un rayonnement à large bande.

16. Composition cosmétique ou dermatologique selon l'une quelconque des revendications 14 et 15, comprenant 0,01 % % à 25 % en poids des particules d'oxyde métallique revêtues.

17. Utilisation des particules d'oxyde métallique revêtues selon l'une quelconque des revendications 1 à 11, pour la stabilisation de filtres UV.

18. Utilisation selon la revendication 17, pour la stabilisation de dibenzoylméthane ou de l'un de ses dérivés.

19. Particules d'oxyde métallique revêtues selon l'une quelconque des revendications 1 à 11, pour une utilisation dans la protection de la peau humaine et/ou de cheveux humains contre un rayonnement UV.
